Europäisches Patentamt

European Patent Office

Office européen des brevets

(19)

(11) Veröffentlichungsnummer: **0 085 894**
**A2**

(12) # EUROPÄISCHE PATENTANMELDUNG

(21) Anmeldenummer: 83100682.0

(22) Anmeldetag: 26.01.83

(51) Int. Cl.³: **A 61 K 7/09**
**A 61 K 7/155**

(30) Priorität: 06.02.82 DE 3204077
27.11.82 DE 3243959

(43) Veröffentlichungstag der Anmeldung:
17.08.83 Patentblatt 83/33

(84) Benannte Vertragsstaaten:
DE FR GB IT NL

(71) Anmelder: Merck Patent Gesellschaft mit beschränkter
Haftung
Frankfurter Strasse 250
D-6100 Darmstadt(DE)

(72) Erfinder: Sandner, Ingo
Walbeweg 3
D-6100 Darmstadt(DE)

(72) Erfinder: Möschl, Gernot
Falltorstrasse 20
D-6108 Weiterstadt 2(DE)

(72) Erfinder: Allardi, Hans-Dieter, Dr.
Ginsterweg 3
D-6101 Seeheim(DE)

(54) Geruchsstabilisierung von Thioglykolat-Lösungen.

(57) Die Erfindung betrifft die Verwendung einer oder mehrerer Substanzen ausgewählt aus der Gruppe bestehend aus Ascorbinsäure, ihren Salzen, 2-Keto-L-gulonsäure, ihren Salzen, mehrwertigen Phenolen und Methenamin zur Geruchsstabilisierung von Lösungen der Thioglykolsäure und/oder ihrer Salze und/oder ihrer Ester.

EP 0 085 894 A2

0085894

Merck Patent Gesellschaft
mit beschränkter Haftung
D a r m s t a d t


Geruchsstabilisierung
von Thioglykolat-Lösungen


Gegenstand der Erfindung ist die Verwendung einer oder mehrerer Substanzen, ausgewählt aus der Gruppe bestehend aus Ascorbinsäure, ihren Salzen, 2-Keto-L-gulonsäure, ihren Salzen, mehrwertigen Phenolen und Methenamin (nachstehend als "Wirkstoffe" bezeichnet) zur Geruchsstabilisierung von Lösungen der Thioglykolsäure und/oder ihrer Salze und/oder ihrer Ester.

Thioglykolsäure und ihre Salze bzw. Ester werden insbesondere in Form ihrer wässerigen Lösungen in der Kosmetik für verschiedene Zwecke verwendet, z.B. Ammoniumthioglykolat zur Herstellung von Kaltwellen, Natrium- und Kaliumthioglykolat zur Entkräuselung, Calciumthioglykolat zur Enthaarung.

Lästig ist bei der Anwendung dieser Lösungen der unangenehme Geruch, der nach Stehen der Lösung auftritt und im wesentlichen auf der Abspaltung von Schwefelwasserstoff beruht.

Es wurde nun überraschend gefunden, daß ein Zusatz eines oder mehrerer der genannten Wirkstoffe z.B. in Mengen zwischen etwa 0,05 und 6 %, vorzugsweise zwischen 0,1 und 4, insbesondere zwischen 0,2 und 2 %, nach kurzer Zeit diesen Geruch wesentlich reduziert. Quantitativ ist nachweisbar, daß dieser Zusatz die Schwefelwasserstoff-Konzentration in der Gasphase über solchen Lösungen erheblich vermindert.

Als Wirkstoffe eignen sich vorzugsweise Ascorbinsäure und 2-Keto-L-gulonsäure, aber auch deren Salze, insbesondere ihre Natrium-, Kalium-, Calcium-, Magnesium- und Ammoniumsalze, ferner Methenamin und mehrwertige insbesondere zwei-, aber auch drei- und höherwertige Phenole, bevorzugt Hydrochinon, weiterhin z.B. Brenzcatechin, Resorcin, Pyrogallol, Phloroglucin, ferner auch mehrwertige Phenole, die weitere Substituenten enthalten, z.B. Gentisinsäure, Gallussäure, deren Salze und Ester, Dichlorophen. Diese Substanzen können zu diesem Zweck in fester Form oder auch in Lösung, vorzugsweise in wässeriger Lösung, verwendet werden.

Zur Geruchsstabilisierung kommen insbesondere wässerige Lösungen der Thioglykolsäure bzw. ihrer Salze und/oder Ester in Frage. Als Salze eignen sich insbesondere das Ammoniumsalz, ferner substituierte Ammoniumsalze, z.B. Mono-, Di- oder Trialkylammonium-, Mono-, Di- oder Trialkanolammonium- wie Mono-, Di- oder Triethanolammonium-, Mono-, Di- oder Triisopropanolammonium-, Tris-hydroxymethylammonium-, ferner Morpholiniumsalze, weiterhin Metall-, vorzugsweise Alkali- oder Erdalkalimetallsalze, z.B. die Natrium-, Kalium-, Lithium-, Calcium-, Strontium- und Magnesiumsalze. Als Ester seien genannt der Methyl-, der Ethyl-, der Glycerinester (= Glycerinmono-thioglykolsäureester) und der Sorbitester (= Sorbit-mono-thioglykolsäureester).

- 4 -

0085894

Die Konzentration der Thioglykolat-Lösungen kann in weiten Grenzen schwanken; sie liegt in der Regel zwischen 1 und 99, vorzugsweise zwischen 4 und 80 %. Die Lösungen können pH-Werte zwischen etwa 2 und etwa 13, insbesondere zwischen 4,5 und 10, vorzugsweise zwischen 5 und 9,5 aufweisen.

Bei der kombinierten Anwendung mehrerer Wirkstoffe können synergistische Effekte auftreten. So wirkt ein gleichzeitiger Zusatz von Ascorbinsäure und Hydrochinon stärker als der jeweilige Zusatz der beiden einzelnen Wirkstoffe allein für sich.

Es wurde weiterhin gefunden, daß eine zusätzliche Zugabe organischer hochmolekularer Stoffe, die üblicherweise als Verdickungsmittel verwendet werden und kolloidale Lösungen bilden können, den geruchsstabilisierenden Effekt der Wirkstoffe verstärkt. Diese hochmolekularen Stoffe sind in Wasser mindestens teilweise löslich (oder kolloid löslich) und zeigen allein keine signifikante geruchsstabilisierende Wirkung. Typische Vertreter dieser Stoffe sind Polyvinylverbindungen wie Polyvinylpyrrolidone, Polyacrylsäuren, Polymethacrylsäuren, Polyvinylalkohole und entsprechende Mischpolymerisate wie Vinylpyrrolidon-Vinylalkohol-, Vinylalkohol-Vinylacetat-, Vinylpyrrolidon-Vinylacetat-Mischpolymerisate; Polyethylenglykole; Cellulosederivate wie Carboxymethylcellulose und deren Salze, insbesondere die Natriumsalze, Methylcellulose, Ethylcellulose, Hydroxyethylcellulose, Hydroxypropylcellulose, Hydroxypropylmethylcellulose; andere Polysaccharide wie Xanthan-Gummi, Galactomannane, Xanthogalactomannane; Alginsäuren und deren Salze; Pektine; Agar-Agar; Tragant; Gummi arabicum; Carrageen.

GSPHA1 H-as

Eine besonders bevorzugte Ausführungsform der Erfindung ist dementsprechend die Verwendung eines oder mehrerer der angegebenen Wirkstoffe zusammen mit einem oder mehreren der angegebenen hochmolekularen Stoffe zur Geruchsstabilisierung. Besonders bevorzugt sind die Kombinationen Ascorbinsäure + Hydrochinon + Polyvinyl-pyrrolidon sowie Ascorbinsäure + Hydrochinon + Carboxy-vinylpolymer (Polyacrylsäure oder Polymethacrylsäure).

Die Thioglykolat-Lösungen können weitere Zusatzstoffe enthalten und können zu den üblichen kosmetischen Zubereitungen (z.B. Lösungen, Emulsionen, Suspensionen, Cremes, Pasten, Gele) verarbeitet werden, die ihrerseits weitere Grund- und/oder Zusatzstoffe enthalten können. Als solche eignen sich z.B. Säuren wie Essigsäure, Milchsäure oder Weinsäure, oder Basen wie Ammoniak oder andere den oben genannten Salzen zugrundeliegende Basen oder Puffer-Salze zur Einstellung des gewünschten pH-Werts; anionische, kationische, nicht-ionische oder amphotere oberflächenaktive Stoffe (Tenside, Emulgatoren, Netzmittel), z.B. Alkylbenzolsulfonate, Alkylnaphthalinsulfonate; Sulfate, Ethersulfate und Sulfonate von Fett-alkoholen, quartäre Ammoniumsalze wie Trimethylcetyl-ammoniumbromid oder Cetylpyridiniumbromid; Fettsäure-amide, -alkanolamide und dialkanolamide, z.B. -diethanolamide; polyoxyethylenierte Säuren, Alkohole oder Alkylphenole; organische Lösungsmittel wie Alkohole (z.B. Ethanol, Isopropanol), Glykole (z.B. Ethylenglykol, 1,2-Propandiol, Glycerin), Glykolether (z.B. 2-Butoxyethanol, Diethylenglykolmonoethylether); anorganische Verdickungsmittel wie Bentonit; Antioxydantien; Chelatisierungsmittel wie Ethylendiamintetraessigsäure (EDTA) und ihre Salze; Fettalkohole, Paraffinkohlenwasserstoffe, Fette, Öle; Polysaccharide; Cystein; Ammoniumtetraborat; Formaldehydsulfoxylat; Harnstoff; antibakteriell wirkende Stoffe wie Bromchlorophen; Farbstoffe; Geruchsstoffe.

GSPHA1 H-as

Die Lösungen und die Zubereitungen können in üblicher Weise durch Mischen der Bestandteile hergestellt werden.

Ammoniumthioglykolat-Lösungen, die Ascorbinsäure enthalten, sind bereits in der DE-OS 14 92 094 beschrieben. Dort finden sich jedoch keinerlei Angaben darüber, daß dieser Stoff geruchsstabilisierend wirkt; nach der Lehre dieser DE-OS wird Ascorbinsäure vielmehr zum Zweck der Wiedererhärtung der (mit der Lösung behandelten) Haut und/oder Haarstrukturen zugesetzt.

Vor- und nachstehend sind alle Prozentangaben als Gewichtsprozente zu verstehen, alle Temperature in $^\circ$C.

Anwendungsbeispiel 1

a)  Man ließ 100 g 59 %ige wässerige vorher mit Stickstoff begaste Ammoniumthioglykolat-Lösung (pH etwa 6,6) in einer braunen 175-ml-Flasche 60 Tage bei 25 - 28$^\circ$ stehen. Danach wurde unter Vermeidung von Erschütterungen mit Hilfe eines Dräger-Röhrchens der Schwefelwasserstoffgehalt in der Gasphase über der Lösung zu ca. 2000 ppm bestimmt.

b)  Vorheriger Zusatz von 1 % Ascorbinsäure führte unter sonst gleichen Bedingungen zu einer Herabsetzung des Schwefelwasserstoffgehalts auf einen Wert von ca. 400 ppm.

Analog wurden nach Zusatz der angegebenen Mengen folgender Substanzen unter sonst gleichen Versuchsbedingungen (wenn nicht anders angegeben) die nachstehenden $H_2S$-Gehalte bestimmt (in ppm, ca.):

| | |
|---|---|
| 1 % Ascorbinsäure bei pH 9 | 100 |
| 1 % 2-Keto-L-gulonsäure | 500 |
| 1 % Hydrochinon | 800 |

GSPHA1 H-as

| | | |
|---|---|---|
| 1 % | Pyrogallol | 950 |
| 1 % | Methenamin | 350 |
| 1 % | Methenamin bei pH 7,5 | 120 (NH$_3$-Geruch!) |

Kombinationen zweier Stoffe:

| | | |
|---|---|---|
| 1 % | Ascorbinsäure + 1 % Hydrochinon | 200 |
| 1 % | Ascorbinsäure + 1 % Pyrogallol | 300 |
| 1 % | Ascorbinsäure + 1 % Methenamin | 200 |
| 1 % | Ascorbinsäure + 1 % Polyvinyl-pyrrolidon[o] | 200 |
| 1 % | Ascorbinsäure + 2 % Vinylpyrrolidon-Vinylacetat-Mischpolymerisat[x] | 150 |
| 1 % | Ascorbinsäure + 1 % Polyethylen-glykol 4000 | 300 |
| 1 % | Ascorbinsäure + 0,2 % Na-Carboxy-methylcellulose | 300 |
| 1 % | Ascorbinsäure + 1 % Bromchlorophen | 250 |
| 1 % | 2-Keto-L-gulonsäure + 1 % Hydrochinon | 250 |
| 1 % | 2-Keto-L-gulonsäure + 1 % Pyrogallol | 200 |
| 1 % | 2-Keto-L-gulonsäure + 1 % Methenamin | 120 |
| 1 % | 2-Keto-L-gulonsäure + 2 % Vinylpyrro-lidon-Vinylacetat-Mischpolymerisat[x] | 150 |
| 1 % | 2-Keto-L-gulonsäure + 0,2 % Carboxy-vinylpolymer[xx] | 100 |
| 1 % | 2-Keto-L-gulonsäure + 0,2 % Na-Carboxy-methylcellulose | 250 |
| 1 % | 2-Keto-L-gulonsäure + 0,5 % Xanthan-Gummi | 250 |
| 1 % | 2-Keto-L-gulonsäure + 1 % Polyethylen-glykol 4000 | 350 |
| 1 % | 2-Keto-L-gulonsäure + 0,2 % Cetyltri-methylammoniumbromid | 400 |

Kombinationen dreier Stoffe:

1 % Ascorbinsäure
+ 1 % Hydrochinon                                    20
+ 1 % Polyvinylpyrrolidon[o)]

1 % Ascorbinsäure
+ 1 % Hydrochinon                                    20
+ 2 % Vinylpyrrolidon-Vinylacetat-
      Mischpolymerisat[x)]

1 % Ascorbinsäure
+ 1 % Hydrochinon                                    20
+ 0,2 % Carboxyvinylpolymer[xx)]

1 % 2-Keto-L-gulonsäure
+ 1 % Hydrochinon                                    40
+ 1 % Carboxyvinylpolymer[xx)]

1 % Ascorbinsäure
+ 0,4 % Polymethacrylsäure[xxx)]                     50
+ 0,2 % Carboxyvinylpolymer[xx)]

1 % Ascorbinsäure
+ 0,5 % Hydrochinon                                  100
+ 0,5 % Pyrogallol

1 % Ascorbinsäure
+ 0,5 % Methenamin                                   100
+ 0,5 % Harnstoff

1 % 2-Keto-L-gulonsäure
+ 1 % Hydrochinon                                    50
+ 1 % Polyvinylpyrrolidon[o)]


[o)] Kollidon® K 30

[x)] Luviskol® VA 64

[xx)] Carbopol® 941

[xxx)] Rohagit® NV

Anwendungsbeispiel 2

Analog Beispiel 1 bestimmte man den Schwefelwasserstoffgehalt über 75%iger wässeriger Thioglykolsäure-glycerinester-Lösung nach 60 Tagen zu ca. 3000 ppm ohne Ascorbinsäure-Zusatz, zu ca. 1500 ppm nach Zusatz von 1 % Ascorbinsäure.

Formulierungsbeispiele:

Beispiel 1:      Ammoniumthioglykolatlösung

| | |
|---|---|
| Thioglykolsäure 99 %ig | 50 % |
| Ascorbinsäure | 1 % |
| Ammoniaklösung 25 %ig | ca. 37 % (bis pH 7) |
| EDTA | 0,3 % |
| Wasser | ad 100 % |

Beispiel 2:      Ethanolammoniumthioglykolatlösung

| | |
|---|---|
| Thioglykolsäure 99 %ig | 40 % |
| Ascorbinsäure | 0,8 % |
| EDTA | 0,4 % |
| Monoethanolamin 98 %ig eisenfrei | ca. 27 % (bis pH 9) |
| Wasser | ad   100 % |

Beispiel 3:      Kaltwell-Lösung (basisch, pH 9,4)

| | |
|---|---|
| Ammoniumthioglykolat-Lösung 59 %ig | 16 g |
| Fettalkoholpolyglykolether | 0,4 g |
| Farbstoff | 0,01 g |
| N-Polyethylenglykol-fettsäureamide | 0,1 g |
| EDTA, Di-Na-Salz | 0,3 g |
| Ascorbinsäure | 1,2 g |
| Wasser | 83 g |
| Ammoniak 10 %ig | bis pH 9,4 |
| Parfümöl | nach Belieben |

GSPHA1 H-as

Beispiel 4:     Kaltwell-Lösung (sauer, pH ca. 6)

| | |
|---|---|
| Thioglykolsäure-glycerinester-Lösung 75%ig | 19 g |
| (oder Thioglykolsäure-sorbitester-Lösung 89%ig | 25 g) |
| Fettalkoholpolyglykolether | 0,4 g |
| Farbstoff | 0,01 g |
| N-Polyethylenglykol-fettsäureamide | 0,1 g |
| EDTA, Di-Na-Salz | 0,3 g |
| Ascorbinsäure | 1,2 g |
| Wasser | 83 g |

Beispiel 5:     Kaltwell-Emulsion

| | |
|---|---|
| Ethanolammoniumthioglykolat-Lösung 67%ig | 20 g |
| Fettalkoholpolyglykolether | 5 g |
| Paraffinöl | 12 g |
| Cetylalkohol | 1 g |
| EDTA, Di-Na-Salz | 0,3 g |
| Cystein | 0,5 g |
| Carboxymethylcellulose, Na-Salz | 0,5 g |
| Ascorbinsäure | 0,7 g |
| Wasser | 60 g |
| Ethanolamin | bis pH 9,3 |
| Parfümöl | nach Belieben |

Beispiel 6:     Entkräuselungspaste

| | |
|---|---|
| Ammoniumthioglykolatlösung 59 %ig | 20 g |
| Cetylstearylalkohol | 20 g |
| Fettalkoholpolyglykolether | 8 g |
| Oleylalkohol | 5 g |
| Butylhydroxyanisol | 0,05 g |
| EDTA | 0,1 g |

| | |
|---|---|
| Farbstoff (z.B. Amaranth) | 0,05 g |
| Ammoniumascorbat | 1,5 g |
| Wasser | 45 g |
| Natronlauge | bis pH 9,5 |
| Parfümöl | nach Belieben |

Beispiel 7:    Enthaarungs-Milch

| | | | |
|---|---|---|---|
| a) | Calciumthioglykolat-trihydrat | | 7,5 g |
| | Calciumhydroxid | | 1,5 g |
| b) | Paraffinöl dickflüssig | | 12 g |
| | $C_8$-$C_{12}$-gesättigte Fettsäuren-Triglycerid | | 3 g |
| | Isopropylmyristat | | 2 g |
| | Cetylalkohol | | 2,5 g |
| | Oleylcetylalkohol-ethoxylat | | 5 g |
| | Siliconöl | | 0,5 g |
| c) | 1,2-Propandiol | | 3 g |
| | Harnstoff | | 4 g |
| | Ascorbinsäure | | 1 g |
| | Wasser | | 58 g |
| | Parfüm | | nach Belieben |

b) und c) werden getrennt auf 75° erhitzt. Danach wird c) unter Rühren zu b) gegeben und unter Rühren abkühlen gelassen. Anschließend wird a) hinzugegeben, parfümiert und schließlich mit Hilfe einer Walzenmühle homogenisiert.

Beispiel 8:    Ammoniumthioglykolatlösung

| | |
|---|---|
| Thioglykolsäure 99%ig | 50 % |
| Ascorbinsäure | 1 % |
| Hydrochinon | 1 % |
| Polyvinylpyrrolidon | 1 % |
| Ammoniaklösung 25%ig | ca. 37 % |
| | (bis pH 7) |
| EDTA | 0,3 % |
| Wasser | ad 100 % |

GSPHA1 H-as

Beispiel 9:     Ethanolammoniumthioglykolatlösung

| | |
|---|---|
| Thioglykolsäure 99 %ig | 40 % |
| Ascorbinsäure | 0,8 % |
| Pyrogallol | 0,4 % |
| Carboxyvinylpolymer | 0,1 % |
| EDTA | 0,4 % |
| Monoethanolamin 98 %ig eisenfrei | ca. 27 % (bis pH 9) |
| Wasser | ad 100 % |

Beispiel 10:     Thioglykolsäureglycerinesterlösung

| | |
|---|---|
| Thioglykolsäureglycerinester 75 %ig | 96,75 % |
| Ascorbinsäure | 1,25 % |
| Hydrochinon | 0,75 % |
| Polyvinylpyrrolidon | 1,25 % |

Beispiel 11:     Kaltwell-Lösung (sauer, pH ca. 6)

| | |
|---|---|
| Thioglykolsäureglycerinester 75 %ig | 20 g |
| Fettalkoholpolyglykolether | 0,4 g |
| Farbstoff | 0,01 g |
| N-Polyethylenglykol-fettsäureamide | 0,1 g |
| EDTA, Di-Na-Salz | 0,3 g |
| Ascorbinsäure | 0,25 g |
| Hydrochinon | 0,15 g |
| Polyvinylpyrrolidon | 0,25 g |
| Wasser | ad 100 g |

Beispiel 12:     Enthaarungs-Creme

| | | |
|---|---|---|
| a) | Calciumthioglykolat-trihydrat | 7,5 g |
| | Calciumhydroxid | 1,5 g |

| b) | Cetylstearylalkohol, emulgierend, DAB 7 | 4,0 g |
| | Cetylstearylalkoholethoxylat | 2,0 g |
| | Pflanzenfettsäuren-partialglyceride | 2,0 g |
| | Paraffin dickflüssig, DAB 7 | 2,0 g |
| | Isopropylmyristat | 1,0 g |
| | 2-Keto-L-gulonsäure | 0,2 g |
| | Hydrochinon | 0,2 g |
| c) | Harnstoff | 3,0 g |
| | 1,2-Propandiol | 5,0 g |
| | Wasser | ad 100 g |
| | Parfüm | nach Belieben |

Die Fettphase b) wird bei etwa 75 $^{\circ}$ geschmolzen und unter Rühren mit der ebenfalls auf etwa 75 $^{\circ}$ gebrachten Lösung c) versetzt. Nach dem Abkühlen der Cremebase werden die Puderbestandteile a) eingerührt. Anschließend wird parfümiert und homogenisiert.

Analog Beispiel 1 - 12 können weitere Formulierungen hergestellt werden, die an Stelle der genannten Zusätze einen Zusatz der übrigen in Anwendungsbeispiel 1 angegebenen Wirkstoffe bzw. Wirkstoff-Kombinationen bzw. Kombinationen von Wirkstoffen und hochmolekularen Stoffen enthalten.

Merck Patent Gesellschaft
mit beschränkter Haftung
D a r m s t a d t


Patentansprüche


1.  Verwendung einer oder mehrerer Substanzen ausgewählt aus der Gruppe bestehend aus Ascorbinsäure, ihren Salzen, 2-Keto-L-gulonsäure, ihren Salzen, mehrwertigen Phenolen und Methenamin zur Geruchsstabilisierung von Lösungen der Thioglykolsäure und/oder ihrer Salze und/oder ihrer Ester.


2.  Verfahren zur Geruchsstabilisierung von Lösungen der Thioglykolsäure und/oder ihrer Salze und/oder ihrer Ester, dadurch gekennzeichnet, daß man eine oder mehrere Substanzen ausgewählt aus der Gruppe bestehend aus Ascorbinsäure, ihren Salzen, 2-Keto-L-gulonsäure, ihren Salzen, mehrwertigen Phenolen und Methenamin zusetzt.


3.  Geruchsstabilisierte Lösungen der Thioglykolsäure und/oder ihrer Salze und/oder ihrer Ester, gekennzeichnet durch einen Gehalt an einer oder mehreren Substanzen ausgewählt aus der Gruppe bestehend aus Ascorbinsäure, ihren Salzen, 2-Keto-L-gulonsäure, ihren Salzen, mehrwertigen Phenolen und Methenamin, mit der Maßgabe, daß eine Lösung, die Ascorbinsäure und/oder ihre Salze enthält, zusätzlich noch mindestens einen der anderen vorstehend genannten Stabilisatoren enthält.


GSPHA1 H-as